# EUROPEAN PATENT SPECIFICATION

(11) **EP 1 732 547 B1**
(45) Date of publication and mention of the grant of the patent: **20.08.2008**
(21) Application number: 05857423.7
(22) Date of filing: 01.04.2005
(51) Int. Cl.: A61K 31/439, A61P 31/18, A61P 37/00, C07D 453/02

(54) **ANTI-HIV QUINUCLIDINE COMPOUNDS**
ANTI-HIV-CHINUKLIDINVERBINDUNGEN
COMPOSES DE QUINUCLIDINE ANTI-VIH

(30) Priority: 05.04.2004 US 559550 P; 22.07.2004 US 590209 P
(43) Date of publication of application: 20.12.2006
(73) Proprietor: Samaritan Pharmaceuticals, Inc., Las Vegas, Nevada 89109 (US); Georgetown University, Washington, DC 20057-1408 (US)
(72) Inventor: LECANU, Laurent, Mclean, VA 22102 (US); GREESON, Janet, Las Vegas, NV 89109 (US); PAPADOPOULOS, Vassilios, North Potomac, MD 20878 (US)
(74) Representative: Lucas, Phillip Brian
(86) International application number: PCT/US2005/011372
(87) International publication number: WO 2006/085890

(56) References cited:
- WO-A-20/04108076
- WO-A-20/05104745

## Description

### Background of the Invention

The global HIV/AIDS epidemic killed more than 3 million people in 2003, and an estimated 5 million acquired the human immunodeficiency virus (HIV) - bringing to 40 million the number of people living with the virus around the world. Despite progress in developing anti-viral regimens, there is not a fully effective therapy for AIDS. Currant therapeutic strategies for AIDS include protease inhibitors, nucleoside analog reverse transcriptase inhibitors, non-nucleoside analog reverse transcriptase inhibitors, fusion inhibitors and also the highly toxic hydroxyurea (Yarchoan R et al. (1986) Lancer 1(8481): 575-580; Richards AD et al (1989) FEBS Lett 247(1): 113-117; Gao WY et al. (1995) Proc Natl Acad Sci USA 92(18): 8333-8337; De Clercq E (1999) Farmaco 54(1-2): 26-45; Williams IG (2003) Int J Clin Pract 57(10): 890-897). Unfortunately emerging resistances due to virus genotype mutations (Cavert W and Balfour HH (2003) Clin Lab Med 23(4):915-928; Gallant JE et al. (2003) Antivir Ther 8(6): 489-506; Olson WC and Maddon PJ (2003) Curr Drug Targets Infect Disord 3(4): 283-294) and serious side-effects are strong limitations to the treatment efficacy.

Currently, there is a need for effective anti-viral agents, including anti-retroviral agents. There is also a need for pharmacological tools for the further study of physiological processes associated with infection.

WO 2005/104745 describes novel bi-aryl 8-azoniabicyclo [3.2.1] octane compounds, pharmaceutical compositions, processes for their preparation and the use thereof in treating muscarinic acetylcholine receptor (mAChR) mediated diseases.

WO 2004/108076 describes the use of an effective amount of an N-benzamidc derivative of a piperazinyl amide or an amino thereof for preventing viral replication by blocking or inhibiting the ability of viruses, such as retroviruses including HIV, to infect mammalian cells in vitro or in vivo.

### Summary of the Invention

The invention provides novel compounds that prevent viral replication by blocking or inhibiting the ability of the viruses, such as retroviruses, including HIV, to infect mammalian cells *in vitro or in vivo*. Thus, the present invention provides a pharmaceutical composition for use in the treatment of a mammal exposed to or afflicted by a virus, by administering to said mammal an effective amount of quinuclidine compound of formula 1: 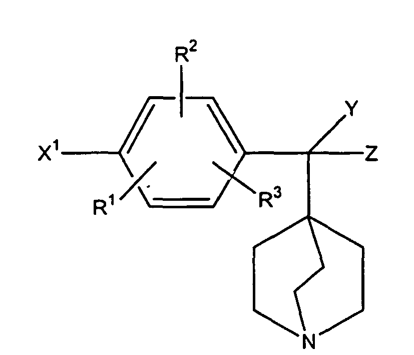 wherein:
a) R¹, R², R³ and R⁵ are individually H, OH, halo, (C₁-C₆)alkyl, (C₁-C₆)alkoxy, (C₃-C₆)cycloalkyl, (C₃-C₆)cycloalkyl((C₁-C₆)alkyl), (C₂-C₆)alkenyl, (C₂-C₆)alkynyl, (C₁-C₆)alkanoyl, halo(C₁-C₆)alkyl, hydroxy(C₁-C₆)alkyl, (C₁-C₆)alkoxycarbonyl; (C₁-C₆)alkylthio or (C₁-C₆)alkanoyloxy; or R¹ and R² together are methylenedioxy;
b) X¹ is NO₂, CN, -N=O, (C₁-C₆)alkylC(O)NH-, oxazolinyl, or N(R⁶)(R⁷) wherein, R⁶ and R⁷ are individually, H, (C₁-C₆)alkyl, (C₂-C₆)alkenyl, (C₃-C₆)cycloalkyl, ((C₁-C₆)alkyl), wherein cycloalkyl optionally comprises 1-2, S, nonperoxide O or N(R⁸), wherein R⁸ is H, O, (C₁-C₆)alkyl, (C₃-C₆)cycloalkyl, (C₃-C₆)cycloalkyl(C₁-C₆)alkyl, phenyl, or benzyl; aryl, aryl(C₁-C₆)alkyl, aryl(C₂-C₆)alkenyl, heteroaryl, heteroaryl(C₁-C₆)alkyl, or R⁶ and R⁷ together with the N to which they are attached form a 5- or 6-membered heterocyclic or heteroaryl ring, optionally substituted with R¹ and optionally comprising 1-2, S, non-peroxide O or N(R⁵);
c) Y and Z are =O, -O(CH₂)ₘO- or -(CH₂)ₘ- wherein m is 2-4, or Y is H and Z is OR⁹ or SR⁹, wherein R⁹ is H or (C₁-C₄)alkyl;
and the pharmaceutically acceptable salts thereof.

Preferably, 1, 2 or 3 of R¹, R² and R³ is H.

Preferably, R⁶ and R⁷ are individually H, (C₁-C₄)alkyl, (C₃-C₆)cycloalkyl, (C₃-C₆)cycloalkyl(C₁-C₆)alkyl or benzyl.

Preferably, X¹ is -N(R⁶)(R⁷).

Preferably, 1 or 2 of R¹, R² or R³ is (C₁-C₆)alkoxy.

Preferably, Z and Y together are =O.

The invention also provides a pharmaceutical composition such as a unit dosage form, comprising a compound of formula I, or a pharmaceutically acceptable salt thereof, in combination with a pharmaceutically acceptable diluent or carrier, which optionally can include one or more anti-HIV agents of one or more of the classes of anti-HIV agents referenced herein above, and can optionally include stabilizers, preservatives, and absorption control agents.

The invention provides a compound of formula I for use in medical therapy (e.g., for use in treating a mammal infected, e.g., with a retrovirus such as HIV), as well as the use of a compound of formula I for the manufacture of a medicament useful for the treatment of infection in a mammal, such as a human.

The invention also provides a method for binding a compound of formula I to mammalian cells to alter the permeability of the cell membranes to infectious agents comprising contacting the cells *in vitro*, with an amount of a compound of formula I effective to interact with, and to alter the properties of the membranes of said cells, e.g., to alter the sterol composition of the cell membranes. Cells comprising a compound of formula I as a ligand bound to receptor sites can be used to measure the selectivity of test compounds for specific receptors on or in cell membranes, or can be used as a tool to identify potential therapeutic agents for the treatment of diseases or conditions dependent on cell membrane permeability, by contacting said agents with said ligand-receptor complexes, and measuring the extent of displacement of the ligand and/or binding of the agent.

The invention provides novel compounds of formula I, as well as, processes and intermediates disclosed herein that are useful for preparing compounds of formula (I) or salts thereof. This includes analogs in which the C(Y)(Z) group is bound to a ring -CH- group of quinuclidine, as well as analogs in which Y and R² are connected by a bond. Many of the compounds of formula I are also useful as intermediates in the preparation of compounds of formula I.

### Brief Description of the Figures

Figure 1 depicts the chemical structure of (4-aminophenyl)(1-aza-bicyclo[2,2,2]oct-4-yl)-methanone or 4-(4-aminobenzoyl)-1-aza-bicyclo[2,2,2]-octane (SP003).
Figure 2 is a graph depicting the inhibitory effect of SP003 on the HIV-1 IIIB strain replication in HeLa cells. Compound was diluted in water or in a formulation (SP003A), dd1 is a known anti-viral compound.
Figure 3 is a graph depicting the inhibitory effect of 24-hour SP003 premedication on the HIV-1 IIIB strain replication in HeLa cells. SP003 were tested in a formulation (SP003A).
Figure 4 is a graph depicting the inhibitory effect of 48-hour SP003 premedication on the HIV-1 IIIB strain replication in HeLa cells.
Figure 5 is a graph depicting the inhibitory effect of SP003 on the multidrug resistant HIV MDR-769 strain replication in HeLa cells. AZT is a known antiviral compound.

### Detailed Description of the Invention

The following definitions are used, unless otherwise described: halo is fluoro, chloro, bromo, or iodo. Alkyl, alkoxy, alkenyl, alkynyl, etc. denote both straight and branched groups; but reference to an individual radical such as "propyl" embraces only the straight chain radical, a branched chain isomer such as "isopropyl" being specifically referred to. Aryl denotes a phenyl radical or an ortho-fused bicyclic carbocyclic radical having nine to ten ring atoms in which at least one ring is aromatic. Heteroaryl encompasses a radical attached via a ring carbon of a monocyclic aromatic ring containing five or six ring atoms consisting of carbon and one to four heteroatoms each selected from the group consisting of non-peroxide oxygen, sulfur, and N(R⁸) wherein R⁸ is absent or is H, O (C₁-C₄)alkyl, phenyl or benzyl, as well as a radical of an ortho-fused bicyclic heterocycle of eight to ten ring atoms derived therefrom, particularly a benz-derivative or one derived by fusing a propylene, trimethylene, or tetramethylene diradical thereto.

It will be appreciated by those skilled in the art that compounds of the invention having a chiral center may exist in and be isolated in optically active and racemic forms. Some compounds may exhibit polymorphism. It is to be understood that the present invention encompasses any racemic, optically-active, polymorphic, or stereoisomeric form, or mixtures thereof, of a compound of the invention, which possess the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase) and how to determine anti-infectious activity using the standard tests described herein, or using other similar tests which are well known in the art.

Specific and preferred values listed below for radicals, substituents, and ranges, are for illustration only; they do not exclude other defined values or other values within defined ranges for the radicals and substituents.

Specifically, (C₁-C₆)alkyl can be methyl, ethyl, propyl, isopropyl, butyl, iso-butyl, sec-butyl, pentyl, 3-pentyl, or hexyl; (C₃-C₆)cycloalkyl can be cyclopropyl, cyclobutyl, cyclopentyl, or cyclohexyl; (C₃-C₆)cycloalkyl(C₁-C₆)alkyl can be cyclopropylmethyl, cyclobutylmethyl, cyclopentylmethyl, cyclohexylmethyl, 2-cyclopropylethyl, 2-cyclobutylethyl, 2-cyclopentylethyl, or 2-cyclohexylethyl; heterocycloalkyl and heterocycloalkylalkyl includes the foregoing cycloalkyl wherein the ring optionally comprises 1-2 S, non-peroxide O or N(R⁸) as well as 2-5 carbon atoms; such as morpholinyl, piperidinyl, piperazinyl, indanyl, 1,3-dithian-2-yl, and the like; (C₁-C₆)alkoxy can be methoxy, ethoxy, propoxy, isopropoxy, butoxy, iso-butoxy, sec-butoxy, pentoxy, 3-pentoxy, or hexyloxy; (C₂-C₆)alkenyl can be vinyl, allyl, 1-propenyl, 2-propenyl, 1-butenyl, 2-butenyl, 3-butenyl, 1,-pentenyl, 2-pentenyl, 3-pentenyl, 4-pentenyl, 1-hexenyl, 2-hexenyl, 3-hexenyl, 4-hexenyl, or 5-hexenyl; (C₂-C₆)alkynyl can be ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, or 5-hexynyl; (C₁-C₆)alkanoyl can be formyl, acetyl, propanoyl or butanoyl; halo(C₁-C₆)alkyl can be iodomethyl, bromomethyl, chloromethyl, fluoromethyl, trifluoromethyl, 2-chloroethyl, 2-fluoroethyl, 2,2,2-trifluoroethyl, or pentafluoroethyl; hydroxy(C₁-C₆)alkyl can be alkyl substituted with 1 or 2 OH groups, such as hydroxymethyl, 1-hydroxyethyl, 2-hydroxyethyl, 1-hydroxypropyl, 2-hydroxypropyl, 3-hydroxypropyl, 1-hydroxybutyl, 4-hydroxybutyl, 3, 4-dihydroxybutyl, 1-hydroxypentyl, 5-hydroxypentyl, 1-hydroxyhexyl, or 6-hydroxyhexyl; (C₁-C₆)alkoxycarbonyl can be methoxycarbonyl, ethoxycarbonyl, propoxycarbonyl, isopropoxycarbonyl, butoxycarbonyl, pentoxycarbonyl, or hexyloxycarbonyl; (C₁-C₆)alkylthio can be methylthio, ethylthio, propylthio, isopropylthio, butylthio, isobutylthio, pentylthio, or hexylthio; (C₂-C₆)alkanoyloxy can be acetoxy, propanoyloxy, butanoyloxy, isobutanoyloxy, pentanoyloxy, or hexanoyloxy; aryl can be phenyl, indenyl, or naphthyl; and heteroaryl can be furyl, imidazolyl, triazolyl, triazinyl, oxazoyl, isoxazoyl, thiazolyl, isothiazoyl, pyrazolyl, pyrrolyl, pyrazinyl, tetrazolyl, pyridyl (or its N-oxide), thienyl, pyrimidinyl (or its N-oxide), 1H-indolyl, isoquinolyl (or its N-oxide) or quinolyl (or its N-oxide).

The term "retrovirus" includes, but is not limited to, the members of the family *retroviridae*, including alpharetroviruses (e.g., avian leukosis virus), betaretroviruses (e.g., mouse mammary tumor virus), gammaretroviruses (e.g., murine leukemia virus), deltaretroviruses (e.g., bovine leukemia virus), epsilonretroviruses (e.g., Walley dermal sarcoma virus), lentiviruses (e.g., HIV-1, HIV-2) and spumaviruses (e.g., human spumavirus).

The present compounds can be prepared by reacting an N-substituted or N-protected phenyl Grignard reagent or lithiated phenyl with 4-cyano-1-aza-bicyclo[2.2.2]octane (4), or 4-cyano-quinuclidine, as shown in Scheme A below, wherein the phenyl Grignard reagent is exemplified by 4-(bis(trimethysilyl))amino-1-phenyl-magnesium bromide.

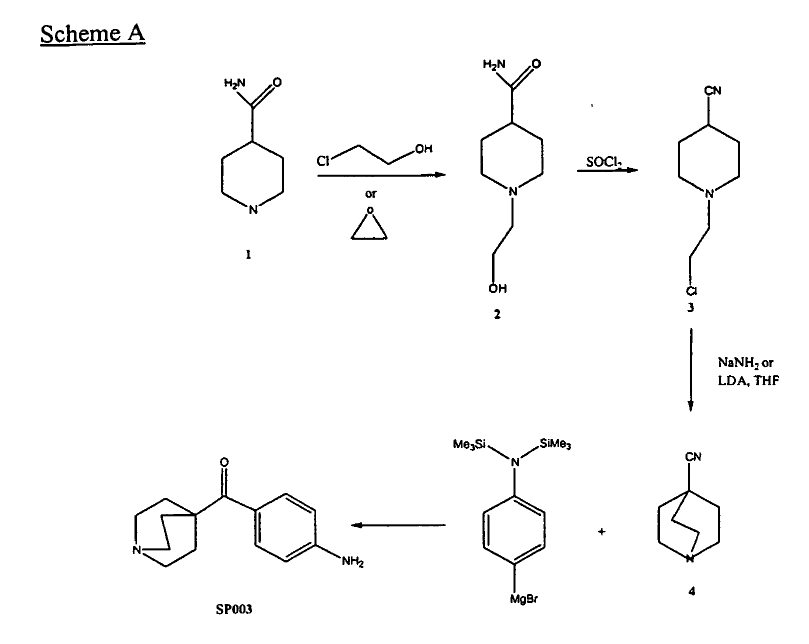

Groups R¹, R² and/or R³ on phenyl that are reactive with Grignard reagents or aryl lithium reagents, such as hydroxy-containing groups can be protected with removable protecting groups such as ethyoxyethyl, THP, (C₁-C₄)₃silyl and the like. Protected HO and hydroxyl alkyl groups can be deprotected, converted into halo, CN, alkoxycarbonyl, alkanoyloxy and alkanoyl by methods known to the art of organic synthesis. Protected amino groups can be deprotected and converted into N(R⁶)(R⁷) by methods known to the art. If necessary the C=O group can be protected and/or reduced during these conversions, then deprotected and reoxidized to C=O. See, for example, F.T. Harrison, Compendium of Organic Synthetic Reactions, Wiley-Interscience, N.Y. (1971); L.F. Fieser et al., Reagents for Organic Synthesis, John Wiley & Sons, Inc., N.Y. (1967), and U.S. Pat. No. 5,411,965.

Thus, a specific value for R¹ in formula I above is H, (C₂-C₄)alkyl, (C₂-C₄)alkoxy or (C₃-C₆)heterocycloalkyl.

A specific value for R² is H.

A specific value for N(R⁶)(R⁷) is amino, diethylamino, dipropylamino, cyclohexylamino, or propylamino, thus a specific value for R³ is NH₂.

Another preferred group of compounds are compounds of formula I which are 2, 3 or 4-(4-substitutedbenzoyl)-1-aza-bicyclo[2,2,2]octanes.

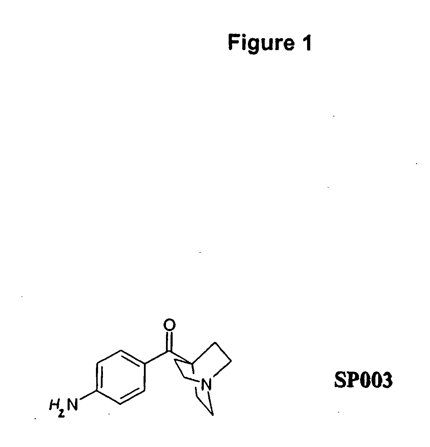

A preferred compound of the invention is SP003 (Fig. 1).

In cases where compounds are sufficiently basic or acidic to form stable nontoxic acid or base salts, administration of the compounds as salts may be appropriate. Examples of pharmaceutically acceptable salts are organic acid addition salts formed with acids which form a physiological acceptable anion, for example, tosylate, methanesulfonate, acetate, citrate, malonate, tartarate, succinate, benzoate, ascorbate, α-ketoglutarate, and α-glycerophosphate. Suitable inorganic salts may also be formed, including hydrochloride, sulfate, nitrate, bicarbonate, and carbonate salts.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium), alkaline earth metal (for example calcium or magnesium) or zinc salts can also be made.

One embodiment of the present invention provides a composition including a compound of formula I and a zinc salt, such as zinc sulfate heptahydrate, wherein ascorbic acid is not preferred in the composition due to a browning effect, e.g., degradation of one or more of the components. In one embodiment, a compound of formula I and a zinc salt, e.g., zinc sulfate heptahydrate, are present in a composition at a ratio of about 27-107 to 1.

The compounds of formula I can be formulated as pharmaceutical compositions and administered to a mammalian host, such as a human patient in a variety of forms adapted to the chosen route of administration, i.e., orally or parenterally, by intravenous, intramuscular, topical or subcutaneous routes, or by inhalation or insufflation.

Thus, the present compounds may be systemically administered, e.g., orally, in combination with a pharmaceutically acceptable vehicle such as an inert diluent or an assimilable edible carrier. They may be enclosed in hard or soft shell gelatin capsules as powders, pellets or suspensions or may be compressed into tablets. For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between 2 to 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, com starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices, such as patches, infusion pumps or implantable depots.

The active compound may also be administered intravenously or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection, infusion or inhalation can include sterile aqueous solutions or dispersions. Sterile powders can be prepared comprising the active ingredient which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate, cellulose ethers, and gelatin.

Sterile injectable solutions are prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the compounds of formula I to the skin are known to the art; for example, see Jacquet et al. (U.S. Pat. No. 4,608,392), Geria (U.S. Pat. No. 4,992,478), Smith et al. (U.S. Pat. No. 4,559,157) and Wortzman (U.S. Pat. No. 4,820,508).

Useful dosages of the compounds of formula I can be determined by comparing their *in vitro* activity, and *in vivo* activity in animal models. Methods for the extrapolation of effective dosages in mice, and other animals, to humans are known to the art; for example, see U.S. Pat. No. 4,938,949.

Generally, the concentration of the compound(s) of formula I in a liquid composition, such as a lotion, will be from 0.1-25 wt-%, preferably from 0.5-10 wt-%. The concentration in a semi-solid or solid composition such as a gel or a powder will be 0.1-5 wt-%, preferably 0.5-2.5 wt-%.

The amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician.

In general, however, a suitable dose will be in the range of from 0.5 to 100 mg/kg, e.g., from 10 to 75 mg/kg of body weight per day, such as 3 to 50 mg per kilogram body weight of the recipient per day, preferably in the range of 6 to 90 mg/kg/day, most preferably in the range of 15 to 60 mg/kg/day.

The compound is conveniently administered in unit dosage form; for example, containing 5 mg to as much as 1-3 g, conveniently 10 to 1000 mg, most conveniently, 50 to 500 mg of active ingredient per unit dosage form.

Ideally, the active ingredient should be administered to achieve peak plasma concentrations of the active compound of from 0.5 to 75 µM, preferably, 1 to 50 µM, most preferably, 2 to 30 µM. This may be achieved, for example, by the intravenous injection of a 0.05 to 5% solution of the active ingredient, optionally in saline. For example, as much as 0.5-3 g of a compound of formula I can be dissolved in 125-500 ml of an intravenous solution comprising, e.g., 0.9% NaCl, and 5-10% glucose. Such solutions can be infused over an extended period of up to several hours, optionally in conjunction with other anti-viral agents, antibiotics, etc. The active ingredient can also be orally administered as a bolus containing 1-100 mg of the active ingredient. Desirable blood levels may be maintained by continuous infusion to provide 0.01-5.0 mg/kg/hr or by intermittent infusions containing 0.4-15 mg/kg of the active ingredient(s).

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

The ability of a compound of the invention to act as an antiviral agent may be determined using pharmacological models which are well known to the art, or using tests described below.

The following illustrate representative pharmaceutical dosage forms, containing a compound of formula I, for therapeutic or prophylactic use in a mammal, such a human.

| (i) Tablet 1 | mg/tablet |
|---|---|
| SP003 | 100.0 |
| Lactose | 77.5 |
| Povidone | 15.0 |
| Croscarmellose sodium | 12.0 |
| Microcrystalline cellulose | 92.5 |
| Magnesium stearate | 3.0 |
| | 300.0 |

| (ii) Tablet 2 | mg/tablet |
|---|---|
| SP003 | 20.0 |
| Microcrystalline cellulose | 410.0 |
| Starch | 50.0 |
| Sodium starch glycolate | 15.0 |
| Magnesium stearate | 5.0 |
| | 500.0 |

| (iii) Capsule | mg/capsule |
|---|---|
| SP003 | 10.0 |
| Colloidal silicon dioxide | 1.5 |
| Lactose | 465.5 |
| Pregelatinized starch | 120.0 |
| Magnesium stearate | 3.0 |
| | 600.0 |

| (iv) Injection 1 (1 mg/ml) | mg/ml |
|---|---|
| SP003 (free base form) | 1.0 |
| Dibasic sodium phosphate | 12.0 |
| Monobasic sodium phosphate | 0.7 |
| Sodium chloride | 4.5 |
| 1.0 N Sodium hydroxide solution (pH adjustment to 7.0-7.5) | q.s. |
| Water for injection | q.s. ad 1 mL |

| (v) Injection 2 (10 mg/ml) | mg/ml |
|---|---|
| SP003 (free base form) | 10.0 |
| Monobasic sodium phosphate | 0.3 |
| Dibasic sodium phosphate | 1.1 |
| Polyethylene glycol 400 | 200.0 |
| 01 N Sodium hydroxide solution (pH adjustment to 7.0-7.5) | q.s. |
| Water for injection | q.s. ad 1 mL |

| (vi) Aerosol | mg/can |
|---|---|
| SP003 | 20.0 |
| Oleic acid | 10.0 |
| Trichloromonofluoromethane | 5,000.0 |
| Dichlorodifluoromethane | 10,000.0 |
| Dichlorotetrafluoroethane | 5,000.0 |

The above formulations may be prepared by conventional procedures well known in the pharmaceutical art.

The invention will be further described by reference to the following detailed examples.

### Example 1. Synthesis of (4-Amino-phenyl)-(1-aza-bicyclo[2.2.2]oct-4-yl)-methanone (SP003)

Compound 4 was synthesized by the method of T. Kanai, Heterocycles (1992) Vol. 34, No. 11, 2137, as shown in Scheme A, hereinabove, N,N-Bis-(trimethylsilyl)-4-bromaniline was purchased from Sigma-Aldrich. Sovlents were purified by standard methods. EI-Mass spectra were recorded on a VG Tribid, Varian CH7 instrument. Thin-layer chromatography (TLC) analyses were performed on silica gel 60 F₂₅₄ on alumina sheets (Merck). NMR-spectroscopy: Bruker AMX300. Chemical shifts are given in ppm.

Under an argon atmosphere, N,N-bis-(trimethylsilyl)-4-bromaniline (2.5 ml, 8.86 mmol) was added dropwise to a suspension of Mg turnings (250 mg, 10.3 mmol) in dry THF (2 ml) at such a rate that consumption of Mg was observed. An aliquot of 1.1 mL of this Grignard reagent was added dropwise to 4 (200 mg, 1.47 mmol) dissolved in dry THF (2 ml). After stirring the solution under reflux for 2 h, ice-cold aqueous HCl (20%, 4 ml) was added. After stirring overnight at ambient temperature the mixture was neutralized with saturated aqueous NaHCO₃ and extracted with dichloromethane. The organic layer was separated, dried over MgSO₄ and concentrated *in vacuo*. The residue was purified by PTLC on silica gel to afford SP003 (44 mg, 0.19 mmol, 13%).

¹H NMR (d-Methanol) δ 7.66 (d, 2 H, 9 Hz), 6.51 (d, 2 H, 9 Hz), 2.86 (m, 6 H), 1.86 (m, 6 H) MS (EI) m/z 230 (M⁺), 174, 135, 110, 96.

### Example 2. Study of the inhibition of HIV-1 IIIB replication on HeLa cells by SP003

### A. Methodology

In order to study the viral replication *in vitro,* the GenPhar AV-Finder^{™}-HIV Drug Discovery Assay was used, a novel technology that consists of two components: (1) a cloned, continuous-passage HeLa cell line containing an HIV-1 tat-activated molecular switch and a Green Fluorescent Protein reporter gene and (2) a recombinant adenovirus (rAd) vector containing the genes for all three of the HIV-1 receptor/co-receptors (CD4, CXCR4, and CCR5) to transduce into HeLa cells and convert them into highly susceptible HIV-1 indicator cells for use in the assay. The indicator cells over-express the HIV-1 receptor genes and are readily infected with any HIV-1 strain or isolate. All HIV-1 strains tested thus far, regardless of co-receptor preference, and all subtypes or classes of HIV-1 will infect these indicator cells. Infected cells fluoresce brightly so that the inhibition of virus replication by potential antiviral drugs can be readily detected and quantified using standard laboratory plate reader technology.

Detector plates are set up at day 1 by adding HeLa cells (3000/well) to the adenovirus AD-3R in DMEM containing CCS in 96-well plates and incubated at 37°C under 95% humidity and 5% CO₂ for 2 days. Without premedication, at day 3, HIV-1 IIIB (200IP/well) and increasing concentrations of SP003 or reference compounds (AZT, ddI, 3TC) were added and incubated overnight. At day 4, the medium was replaced by fresh medium containing the corresponding concentration of the compounds of interest. The infectivity was assessed by measuring the fluorescence on each well at day 7 (λₑₘᵢₛ=485 nm; λ_{exc}=520 nm). With 24 hours pre-medication, increasing concentrations of SP003 or reference compounds (AZT, ddI, 3TC) were added at day 3 and incubated overnight. At day 4, HIV-1 IIIB (200IP/well) and increasing concentrations of SP003 or reference compounds (AZT, ddI, 3TC) were added and incubated overnight. At day 5, the medium was replaced by fresh medium containing the corresponding concentration of compounds of interest and the infectivity was assessed by measuring the fluorescence on each well at day 8. Results are expressed as percentage of inhibition of the viral replication.

Following the above described cell treatment protocol, the levels of cellular 3-(4,5-dimehtylthiazol-2-yl)-2,5-diphenyl tetrazolium bromide (MTT) reduction, a measure of mitochondrial integrity, were determined in order to examine whether the compounds tested were cytotoxic.

(4-amino-phenyl)-(1-aza-bicyclo[2.2.2]oct-4-yl)-methanone (Fig. 1) was used either alone dissolved in water (SP003) or in an aqueous formulation (SP003A) containing zinc sulfate heptahydrate and ascorbic acid at the ratio of 26.6/1/1.6 (for example 200 mg SP003 with 7.5 mg of zinc sulfate heptahydrate and 12.5 mg of ascorbic acid; Xu, J. et al., J. Pharmacol. Exper. Ther., (2003) 307:1148-1157.

### B. Results

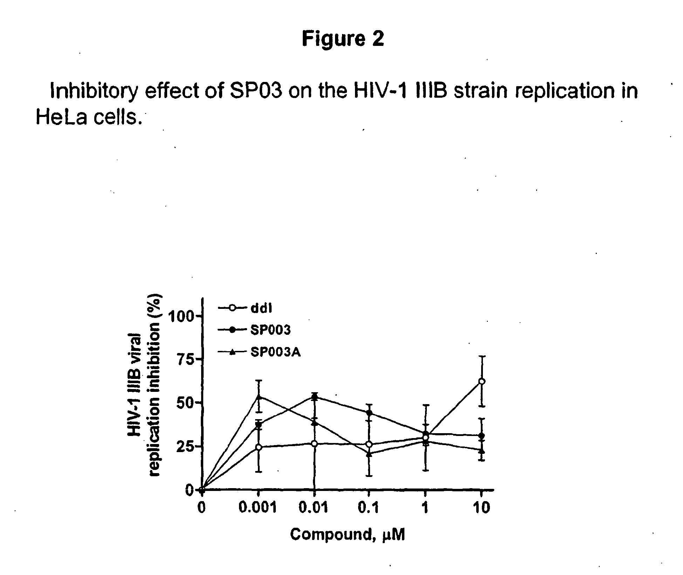

### 1. Effect on HIV-1 IIB viral replication. No pre-medication (Fig. 2)

The structure of the compound (4-amino-phenyl)-(1-azabicyclo[2,2,2]oct-4-yl)-methanone (SP003) is shown in Fig. 1. The compounds was dissolved in water or when indicated in the formulation containing zinc sulfate heptahydrate, ascorbic acid and sodium benzoate. (SP003A). SP003 inhibited the HIV-1 IIIB viral replication with a higher efficiency than the classical antiviral agent ddI when given at concentrations up to 1 µM (Fig. 2). SP01A also inhibited viral replication at very low concentrations (Fig. 2). Interestingly SP003 and SP003A at all concentrations tested, up to 100 µM were devoid of cell toxicity as assessed by the MTT cytotoxicity assay in contrast to the classical antiviral agents ddI, AZT and 3TC which showed toxicity with an IC50 of 89 and 161 and 71 µM concentrations, respectively. Thus, for future experiments and in order to be able to accurately compare the antiviral properties of the compounds under investigation to that of classical antiviral agents, concentrations ranging from pM up to 10 µM were evaluated.

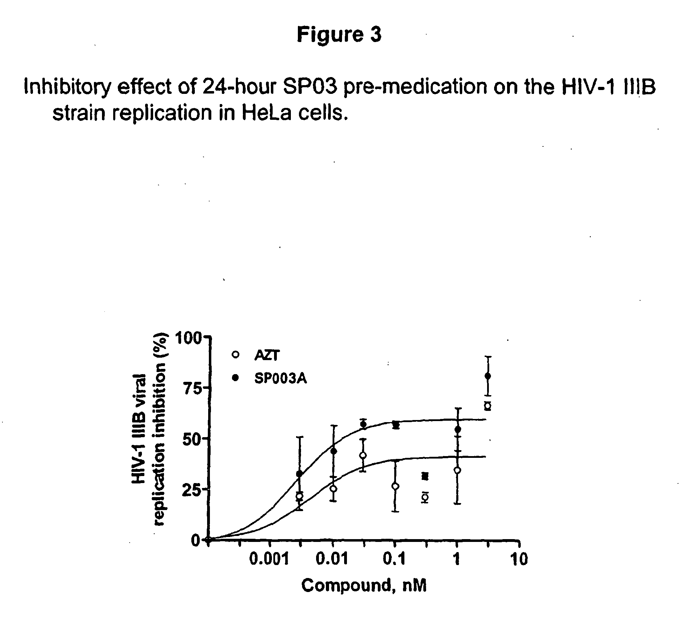

### 2. Effect of HIV-1 IIB viral replication. Effects of 24 hours pre-medication (Fig. 3)

In this study SP003A was used. After 24 hours pre-medication, SP003A displayed a better efficacy than AZT on viral replication (Fig. 3).

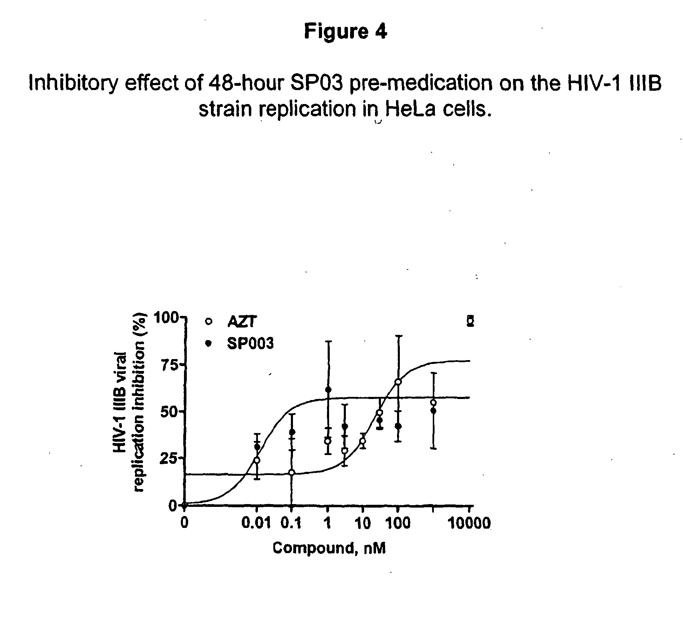

### 3. Effect on HIV-1 IIB viral replication. Effects of 48 hours pre-medication (Fig. 4)

48 hours pretreatment with SP003 inhibited by 50% HIV replication with and IC50 of 0.03 nM (Fig. 4). Under the same protocol AZT inhibited the HIV replication in a dose-dependent manner with an IC50 of 30 nM.

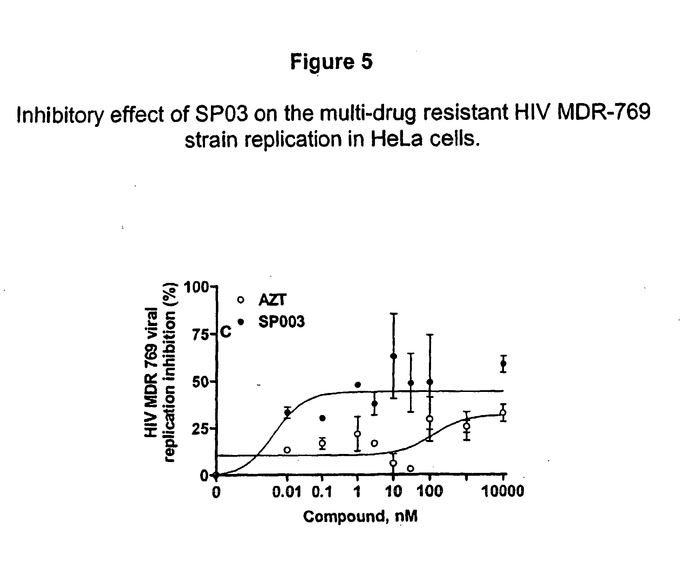

### 4. Effect on HIV MDR 769 viral replication. Effects without pre-medication (Fig. 5)

As expected AZT was not effective in inhibiting the HIV MDR 769 strain replication (Fig. 5). SP003 inhibited by 60% the HIV MDR 769 viral replication with an IC50 of 0.01 nM (Fig. 5).

These examples demonstrate the ability of the quinuclidine compound, (4-amino-phenyl)-)1-aza-bicyclo[2,2,2]oct-4-yl)-methanone (SP003) to reduce HIV-1 IIB replication in human cells with an efficacy higher than AZT or ddI. In an experimental protocol without pre-medication, an inhibition of HIV-1 IIIB replication by this compound was observed of up to 50% with concentrations in the nanomolar range and there was not a major difference between the compound dissolved in water compared to that dissolved in the zinc sulfate heptahydrate and ascorbic acid solution (SP003A).

In order to assess whether the virus was the direct target of the compound or another mechanism may be mediating the effect of this compound on viral replication, the HeLa cells were pre-medicated for 24 hours with SP003 in a formulation containing zinc sulfate heptahydrate and ascorbic acid before the virus was added. Interestingly, the effect obtained was much stronger than without pre-medication and with concentrations in the picomolar range. The curve plateau was at more than 60% inhibition for SP003A whereas it was around 40% for AZT. Preincubation of the cells with the compound under investigation for a 48 hours time period had also a pronounced effect. This difference in efficacy displayed after pre-medication versus no pre-medication suggests that the compounds of formula I may not directly target the virus, but more likely modify the sensitivity of the cells to the virus entry, rendering them more resistant to infection. In contrast to the classical anti-viral agents, such AZT and ddI, SP003 should be effective in blocking the HIV MDR 769 virus replication, due to reduced infectivity of the cells. Indeed, although AZT was ineffective in blocking HIV MDR 769 virus replication, SP003 effectively blocked the replication of the virus/infectivity of the cells.

In conclusion, the data herein demonstrate that (4-amino-phenyl)-(1-azabicyclo[2.2.2]oct-4-yl)-methanone (SP003) provides a new anti-retroviral therapy that will be efficacious either alone or in combination with HAART and mega HAART therapies. These results indicate that this compound acts most likely on the cells by increasing their resistance to virus entry rather than acting directly on the virus itself. Although the mechanism of action is not fully understood, acting on the host cells, rather than on the virus, is expected to lower the rate of emergence of resistant strains and therefore to increase the efficacy of anti-retroviral therapies.

The invention has been described with reference to various specific and preferred embodiments and techniques. However, it should be understood that many variations and modifications may be made while remaining within the scope of the invention.

## Claims

1. A quinuclidine compound of formula I: 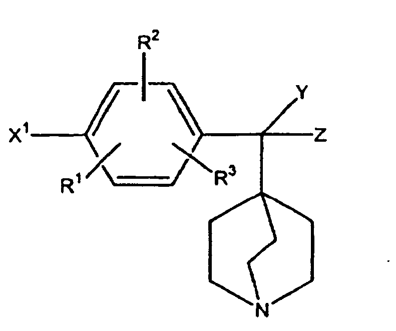 wherein:
a) R¹, R², R³ and R⁵ are individually H, OH, halo, (C₁-C₆)alkyl, (C₁-C₆)alkoxy, (C₃-C₆)cycloalkyl, (C₃-C₆)cycloalkyl((C₁-C₆)alkyl), (C₂-C₆)alkenyl, (C₂-C₆)alkynyl, (C₁-C₆)alkanoyl, halo(C₁-C₆)alkyl, hydroxy(C₁-C₆)alkyl, (C₁-C₆)alkoxycarbonyl; (C₁-C₆)alkylthio or (C₁-C₆)alkanoyloxy; or R¹ and R² together are methylenedioxy;
b) X¹ is NO₂, CN, -N=O, (C₁-C₆)alkylC(O)NH-, oxazolinyl, or N(R⁶)(R⁷) wherein, R⁶ and R⁷ are individually, H, (C₁-C₆)alkyl, (C₂-C₆)alkenyl, (C₃-C₆)cycloalkyl, ((C₁-C₆)alkyl), wherein cycloalkyl optionally comprises 1-2, S, nonperoxide O or N(R⁸), wherein R⁸ is H, O, (C₁-C₆)alkyl, (C₃-C₆)cycloalkyl, (C₃-C₆)cycloalkyl(C₁-C₆)alkyl, phenyl, or benzyl; aryl, aryl(C₁-C₆)alkyl, aryl(C₂-C₆)alkenyl, heteroaryl, heteroaryl(C₁-C₆)alkyl, or R⁶ and R⁷ together with the N to which they are attached form a 5- or 6-membered heterocyclic or heteroaryl ring, optionally substituted with R¹ and optionally comprising 1-2, S, non-peroxide O or N(R⁵);
c) Y and Z taken together are =O, -O(CH₂)ₘO- or -(CH₂)ₘ- wherein m is 2-4, or Y is H and Z is OR⁹ or SR⁹, wherein R⁹ is H or (C₁-C₄)alkyl;
and the pharmaceutically acceptable salts thereof.

2. The compound of claim 1, wherein X¹ is N(R⁶)(R⁷).

3. The compound of claim 1 or claim 2, wherein X¹ is NH₂.

4. The compound of any one of claims 1 to 3 wherein 1, 2 or 3 of R¹, R² & R³ is H.

5. The compound of any one of claims 1-3, wherein 1 or 2 of R¹, R² or R³ is H or (C₁-C₆)alkoxy, preferably (C₁-C₃)alkoxy.

6. The compound of any one of claims 1 to 5 wherein R⁶ & R⁷ are individually H, (C₁-C₄) alkyl, (C₃-C₆) cycloalkyl, (C₃-C₆) cycloalkyl (C₁-C₆) alkyl or benzyl.

7. The compound of any one of claims 1-6, wherein Y and Z together are =O.

8. The compound of any one of claims 1-6, wherein Y is OH and Z is H.

9. The compound of any one of claims 1-8, wherein R¹, R² and R³ are H.

10. The compound of claim 1, which is [4-amino-phenyl)-(1-aza-bicyclo[2,2,2]oct-4-yl) methadone.

11. A pharmaceutical composition comprising a therapeutically effective amount of a compound according to any one of claims 1 to 10 together with a pharmaceutically acceptable carrier.

12. The composition of claim 11, wherein the carrier is a liquid, such as a solution, suspension or gel.

13. The composition of claim 11, wherein the carrier is a solid.

14. The composition of any one of claims 11-13, wherein the carrier comprises a zinc salt, preferably zinc sulfate heptahydrate.

15. The composition of any one of claims 11-14, wherein the composition is in a unit dosage form.

16. The composition according to any one of claims 11 to 15 also comprising one or more anti-HIV agents.

17. The composition according to any one of claims 11-16 which is in a form suitable for administration orally, parentally, by inhalation or insufflation.

18. Use of a compound of formula I according to any one of claims 1 to 10 in the preparation of a medicament for treating a mammal exposed to or afflicted by a virus.

19. The use of claim 18, wherein the virus is a retrovirus.

20. The use of claim 19, wherein the retrovirus is HIV.

21. A method for preparing a compound of formula I as claimed in claim 1 by reacting an N-substituted or N-protected phenyl Grignard reagent or lithiated phenyl with 4-cyano-1-aza-bicyclo [2.2.2] octane or 4-cyano-quinclidine.

## Patentansprüche

1. Chinuclidinverbindung der Formel I: 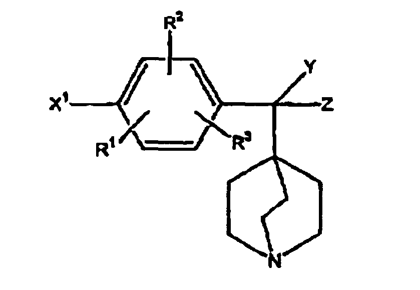 worin:
a) R¹, R², R³ und R⁵ einzeln H, OH, ein Halogen, (C₁-C₆)Alkyl, (C₁-C₆)Alkoxy, (C₃-C₆)Cycloalkyl, (C₃-C₆)Cycloalkyl((C₁-C₆)alkyl), (C₂-C₆)Alkenyl, (C₂-C₆)Alkinyl, (C₁-C₆)Alkanoyl, Halogen(C₁-C₆)alkyl, Hydroxy(C₁-C₆)alkyl, (C₁-C₆)alkoxycarbonyl; (C₁-C₆)Alkylthio oder (C₁-C₆)Alkanoyloxy sind oder R¹ und R² zusammen Methylendioxy sind;
b) X¹ ist: NO₂, CN, -N=O, (C₁-C₆)AlkylC(O)NH-, Oxazolinyl oder N(R⁶)(R⁷), wobei R⁶ und R⁷ einzeln H, (C₁-C₆)Alkyl, (C₂-C₆)Alkenyl, (C₃-C₆)Cycloalkyl, ((C₁-C₆)Alkyl) sind, wobei Cycloalkyl gegebenenfalls 1 bis 2 S, nicht als Peroxid vorkommendes O oder N(R⁸) umfaßt, wobei R⁸ gleich H, O, (C₁-C₆)Alkyl, (C₃-C₆)Cycloalkyl, (C₃-C₆)Cycloalkyl(C₁-C₆)alkyl, Phenyl oder Benzyl ist; Aryl, Aryl(C₁-C₆)alkyl, Aryl(C₂-C₆)alkenyl, Heteroaryl, Heteroaryl(C₁-C₆)alkyl ist, oder R⁶ und R⁷ zusammen mit dem N, an das sie gebunden sind, einen 5- oder 6-gliedrigen heterocyclischcn oder Heteroarylring bilden, der gegebenenfalls mit R¹ substituiert ist und gegebenenfalls 1 bis 2 S, nicht als Peroxid vorkommendes O oder N(R⁵) umfaßt;
c) Y und Z zusammen =O, -O(CH₂)ₘO- oder -(CH₂)ₘ- sind, wobei m gleich 2 bis 4 ist, oder Y gleich H ist und Z gleich OR⁹ oder SR⁹ ist, wobei R⁹ gleich H oder (C₁-C₄)Alkyl ist;
und die pharmazeutisch akzeptablen Salze davon.

2. Verbindung nach Anspruch 1, wobei X¹ gleich N(R⁶)(R⁷) ist.

3. Verbindung nach Anspruch 1 oder Anspruch 2, wobei X¹ gleich NH₂ ist.

4. Verbindung nach einem der Ansprüche 1 bis 3, wobei 1, 2 oder 3 der Reste R¹, R² und R³ gleich H sind.

5. Verbindung nach einem der Ansprüche 1 bis 3, wobei 1 oder 2 der Reste R¹, R² oder R³ gleich H oder (C₁-C₆)Alkoxy, vorzugsweise (C₁-C₃)Alkyoxy sind.

6. Verbindung nach einem der Ansprüche 1 bis 5, wobei R⁶ und R⁷ einzeln H, (C₁-C₄)Alkyl, (C₃-C₆)Cycloalkyl, (C₃-C₆)Cycloalkyl(C₁-C₆)alkyl oder Benzyl sind.

7. Verbindung nach einem der Ansprüche 1 bis 6, wobei Y und Z zusammen =O sind.

8. Verbindung nach einem der Ansprüche 1 bis 6, wobei Y gleich OH ist und Z gleich H ist.

9. Verbindung nach einem der Ansprüche 1 bis 8, wobei R¹, R² und R³ gleich H sind.

10. Verbindung nach Anspruch 1, welche [4-Aminophenyl)-(1-azabi-cyclo[2.2.2]oct-4-yl)methanon ist.

11. Pharmazeutische Zusammensetzung, die eine therapeutisch wirksame Menge einer Verbindung nach einem der Ansprüche 1 bis 10 zusammen mit einem pharmazeutisch akzeptablen Träger umfaßt.

12. Zusammensetzung nach Anspruch 11, wobei der Träger eine Flüssigkeit, wie eine Lösung, Suspension oder ein Gel ist.

13. Zusammensetzung nach Anspruch 11, wobei der Träger ein Feststoff ist.

14. Zusammensetzung nach einem der Anspruch 11 bis 13, wobei der Träger ein Zinksalz, vorzugsweise Zinksulfatheptahydrat umfaßt.

15. Zusammensetzung nach einem Ansprüche 11 bis 14, wobei die Zusammensetzung in Form einer Dosierungseinheit vorliegt.

16. Zusammensetzung nach einem der Ansprüche 11 bis 15, die ebenfalls ein oder mehrere Mittel gegen HIV umfaßt.

17. Zusammensetzung nach einem der Ansprüche 11 bis 16, die in einer Form vorliegt, die für die orale, parenterale, durch Inhalation oder Einblasen erfolgende Verabreichung geeignet ist.

18. Verwendung einer Verbindung der Formel I nach einem der Ansprüche 1 bis 10 bei der Herstellung eines Medikamentes zur Behandlung eines Säugers, der einem Virus ausgesetzt ist oder an diesem leidet.

19. Verwendung nach Anspruch 18, wobei der Virus ein Retrovirus ist.

20. Verwendung nach Anspruch 19, wobei der Retrovirus HIV ist.

21. Verfahren zur Herstellung einer Verbindung der Formel I nach Anspruch 1 durch Reaktion eines Grignard-Reagenz mit N-substituiertem oder N-geschütztem Phenyl oder mit Lithium behandeltem Phenyl mit 4-Cyano-1-azabicyclo[2.2.2]octan oder 4-Cyanochinuclidin.

## Revendications

1. Composé quinuclidine de formule I : 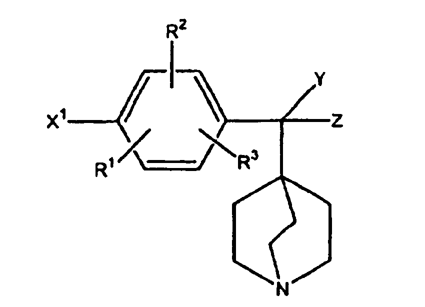 dans laquelle :
a) R¹, R², R³ et R⁵ représentent chacun H, OH, halogéno, alkyle en C₁ à C₆, alcoxyle en C₁ à C₆, cycloalkyle en C₃ à C₆, (cycloalkyle en C₃ à C₆)-(alkyle en C₁ à C₆), alcényle en C₂ à C₆, alcynyle en C₂ à C₆, alcanoyle en C₁ à C₆, halogénoalkyle en C₁ à C₆, hydroxyalkyle en C₁ à C₆, (alcoxy en C₁ à C₆)-carbonyle ; alkylthio en C₁ à C₆ ou alcanoyloxy en C₁ à C₆ ; ou R¹ et R² forment ensemble un méthylènedioxy ;
b) X¹ représente NO₂, CN, -N=O, un groupe (alkyle en C₁ à C₆)-C(O)NH-, oxazolinyle ou N(R⁶)(R⁷) où, R⁶ et R⁷ représentent chacun H, alkyle en C₁ à C₆, alcényle en C₂ à C₆, cycloalkyle en C₃ à C₆, alkyle en C₁ à C₆, où le groupe cycloalkyle comprend éventuellement 1 ou 2 atomes de S, O, non-peroxyde ou N(R⁸), où R⁸ représente H, 0, alkyle en C₁ à C₆, cycloalkyle en C₃ à C₆, (cycloalkyle en C₃ à C₆)-(alkyle en C₁ à C₆), phényle ou benzyle ; aryle, aryl-(alkyle en C₁ à C₆), aryl-(alcényle en C₂ à C₆), hétéroaryle, hétéroaryl-(alkyle en C₁ à C₆), ou R⁶ et R⁷ forment, conjointement avec l'atome d'azote auquel ils sont liés, un hétérocycle ou groupe hétéroaryle à 5 ou 6 éléments éventuellement substitué par R¹ et comprenant éventuellement 1 ou 2 S, O non-peroxyde ou N(R⁵) ;
c) Y et Z forment ensemble =0, -O(CH₂)ₘO- ou -(CH₂)ₘ-, où m vaut 2 à 4, ou Y représente H et Z est OR⁹ ou SR⁹, où R⁹ est H ou alkyle en C₁ à C₄ ; et ses sels acceptables sur le plan pharmaceutique.

2. Composé selon la revendication 1, dans lequel X¹ est N(R⁶)(R⁷).

3. Composé selon la revendication 1 ou 2, dans lequel X¹ est NH₂.

4. Composé selon l'une quelconque des revendications 1 à 3, dans lequel 1, 2 ou 3 de R¹, R² et R³ sont H.

5. Composé selon l'une quelconque des revendications 1 à 3, dans lequel 1 ou 2 de R¹, R² ou R³ représentent H ou alcoxyle en C₁ à C₆, de préférence alcoxyle en C₁ à C₃.

6. Composé selon l'une quelconque des revendications 1 à 5, dans lequel R⁶ et R⁷ représentent chacun H, alkyle en C₁ à C₄, cycloalkyle en C₃ à C₆, (cycloalkyle en C₃ à C₆)-(alkyle en C₁ à C₆) ou benzyle.

7. Composé selon l'une quelconque des revendications 1 à 6, dans lequel Y et Z forment ensemble =O.

8. Composé selon l'une quelconque des revendications 1 à 6, dans lequel Y est OH et Z est H.

9. Composé selon l'une quelconque des revendications 1 à 8, dans lequel R¹, R² et R³ représentent H.

10. Composé selon la revendication 1, qui est la (4-aminophényl)-(1-azabicyclo[2.2.2]oct-4-yl)méthanone.

11. Composition pharmaceutique comprenant une quantité thérapeutiquement efficace d'un composé selon l'une quelconque des revendications 1 à 10 conjointement avec un véhicule pharmaceutiquement acceptable.

12. Composition selon la revendication 11, dans laquelle le véhicule est un liquide tel qu'une solution, une suspension ou un gel.

13. Composition selon la revendication 11, dans laquelle le véhicule est un solide.

14. Composition selon l'une quelconque des revendications 11 à 13, dans laquelle le véhicule comprend un sel de zinc, de préférence du sulfate de zinc heptahydraté.

15. Composition selon l'une quelconque des revendications 11 à 14, dans laquelle la composition est une forme galénique unitaire.

16. Composition selon l'une quelconque des revendications 11 à 15, comprenant également un ou plusieurs agents anti-VIH.

17. Composition selon l'une quelconque des revendications 11 à 16, qui est sous une forme appropriée à une administration par voie orale, par voie parentérale, par inhalation ou par insufflation.

18. Utilisation d'un composé de formule I selon l'une quelconque des revendications 1 à 10 pour la préparation d'un médicament destiné à traiter un mammifère exposé à ou affecté par un virus.

19. Utilisation selon la revendication 18, dans laquelle le virus est un rétrovirus.

20. Utilisation selon la revendication 19, dans laquelle le rétrovirus est le VIH.

21. Procédé de préparation d'un composé de formule I selon la revendication 1 par réaction d'un réactif de Grignard phényle N-substitué ou N-protégé ou un phényle lithié avec du 4-cyano-1-aza-bicyclo[2.2.2]octane ou de la 4-cyanoquinuclidine.
